# EUROPEAN PATENT APPLICATION

(11) **EP 4 530 600 A1**
(43) Date of publication of application: **02.04.2025**
(21) Application number: 23200354.1
(22) Date of filing: 28.09.2023
(51) Int. Cl.: G01N 1/10, G01N 1/12, G01N 1/14, G01N 1/20, G01N 33/18

(54) **PROCESS WATER SAMPLING IMMERSION PROBE**

(71) Applicant: Hach Lange GmbH, 14163 Berlin (DE)
(72) Inventor: Weber, Roman, 14163 Berlin (DE); Draeger, Hartmut, 14163 Berlin (DE); Steinhauer, Frank, 14163 Berlin (DE); de Heij, Bas, 14163 Berlin (DE); Kussmann, Michael, 14163 Berlin (DE)
(74) Representative: terpatent PartGmbB

(57) **Abstract**

The invention relates to a process water sampling immersion probe (10) comprising
a filter module (20) comprising a filter screen (24, 24') and
a cleaning air generator (40) arranged vertically adjacent to the vertical lower end (d24) of the filter screen (24, 24'),
wherein the cleaning air generator (40) comprises a bubble generator housing (41) with an air exit opening (51, 52) having a non-horizontal opening plane (p54),
wherein the air exit opening (51, 52) widens vertically downwardly so that the air exit opening top width (wt54) at the openings top end (t54) is smaller than the air exit opening down width (wd54) at the openings lower end (d54).

## Description

The present invention relates to a process water sampling immersion probe for continuously filtering a water sample from wastewater.

Stationary process water sampling immersion probes are used as a part of a process water analysis arrangement for analyzing one or more analytes in water, for example in wastewater in a wastewater tank being a part of a wastewater treatment plant.

A typical stationary water sampling immersion probe is disclosed in DE 10 2004 037 226 B3. The water sampling immersion probe is provided with two filter screens each lying in a filter screen plane being inclined with respect to the terrestrial vertical axis. At the lower end of every filter screen, a horizontal row of several air exit openings is provided through which pumped air exits and thereby generates a cleaning air bubble curtain along the assigned filter screen. The air exit openings can become overgrown and clogged by the wastewater substances so that the cleaning quality of the air curtain decreases continuously. Therefore, the air exit openings must be manually cleaned from time to time which is cumbersome. The air pump providing the pressurized cleaning air must run continuously to reduce the clogging tendency and the temporal clogging rate.

It is an object of the invention to improve the handling and the performance of a process water sampling immersion probe.

This object is solved with a process water sampling immersion probe with the features of main claim 1.

The process water sampling immersion probe for continuously filtering a water sample from water, preferably from wastewater, comprises a filter module with a filter screen for filtering the water which is sucked through the filter screen and is pumped to a land-based analyzer unit where the analyte is determined quantitatively. The process water sampling immersion probe also comprises a cleaning air generator arranged vertically adjacent to the vertical lower end of the filter screen. The cleaning air generator comprises a bubble generator housing with at least one air exit opening, the air exit opening having a non-horizontal opening plane.

Preferably, at least two air exit openings are provided at the bubble generator housing. The cleaning air pumped to the bubble generator housing exits through the air exit opening so that air bubbles are generated distally of the air exit opening which rise along the distal surface of the filter screen with a vertical component. The opening plane of the air exit opening is not lying in a horizontal plane but is lying in a more vertical inclined plane or even in a perfectly vertical plane.

The air exit opening has a vertical top end and a vertical lower end, whereas the air exit opening's horizontal top width at the top end of the air exit opening is smaller than the air exit opening's low width at the lower end of the air exit opening. The width of the air exit opening is the distance from a point of an opening edge to the horizontally opposite point of the opposite opening edge.

Generally, it is not possible to avoid any clogging of the air exit opening in particular in wastewater applications. Since the air exit opening is horizontally widening in the downward direction, the pressure difference between the pressurized cleaning air inside the bubble generator housing and the wastewater outside of the bubble generator housing causes a braking force with respect to a clogging structure, whereas the total breaking force proportionally increases with the horizontal width of the air exit opening. Even if the clogging structure should be relatively strong, the clogging structure will break at a relatively low location of the air exit opening where the air exit width is relatively large. Another effect of the downwardly extending air exit opening is the fact that the clogging structure is continuously eroded by the cleaning air flow in the horizontal center of the air exit opening, which has an indentation effect in the center of the clogging structure. Whereas the vertical position of the true air exit location can vary dependent on the vertical extension of the clogging structure in the air exit opening, the horizontal position of the true air exit location does not substantially change and is relatively precisely defined in the horizontal opening center independent of the vertical extension of the clogging structure.

As a result, the structure of the air bubble carpet is not substantially affected by clogging of the air exit openings. Since the clogging of the air exit opening is not substantially affecting the structure and the quality of the cleaning air bubble carpet, the cleaning air pump does not need to be continuously activated with maximum pumping performance, so that the lifetime of the cleaning air pump is extended.

The process water sampling immersion probe has been tested in a field test in a tank of a waste water treatment plant in the year 2023. Even after 160 days without any manual cleaning of the water sampling immersion probe, the cleaning air generator worked without any relevant deterioration of the quantity and the quality of the generated air bubble curtain. As a result, the pumping performance of continuously pumping the filtered waste water from the process water sampling immersion probe to the land-based analyzer station had only been decreased from 1600 ml/h to 1400 ml/h. As a practical consequence, the manual cleaning interval can be substantially extended by at least a factor 2.0.

Preferably, the air exit opening continuously widens between the opening's top end and the opening's lower end of the air exit opening. The width of the air exit opening widens not stepwisely but continuously from the vertical top end to the vertical low end of the air exit opening.

Preferably, the air exit opening is substantially inverse V-shaped whereas the lateral edges of the air exit opening are not necessarily linear but can be curved so that the opening's edges of the air exit opening define a progressive vertical increase of the opening's width in vertical downward direction. More preferably, the average total opening angle of the inverse V-shaped air exit opening is more than 15°, more preferably is more than 30°.

Preferably, at least two air exit openings are provided for one filter screen. More preferably, two parallel filter screens are provided and at least two air exit openings are provided for each filter screen, respectively.

Preferably, the bubble generator housing has an inverse cup -like structure with a housing opening having a horizontal housing opening plane. The air exit opening extends into the horizontal housing opening so that the air exit opening has no opening edge closing the low side of the air exit opening. The lower end of the air exit opening extends seamlessly into the horizontal housing opening. As a result, the air exit openings are easily accessible from the bottom side of the bubble generator housing and can easily be mechanically cleaned, either manually or automatically.

Preferably, the bubble generator housing comprises a substantially vertical side wall comprising the air exit openings. As a result, the lateral distance of the true air exit defined by the clogging structure remains always the same with respect to the general plane of the filter screen so that the air bubbles leaving the cleaning air generator always remain close to the filter screen independent of the expansion of the clogging structure in the air exit opening.

Preferably, a guidance arrangement with a vertical guidance wall is provided for guiding the air bubble curtain. The guidance wall is distally horizontally spaced apart from the corresponding filter screen and thereby defines a vertical air bubble channel between the filter screen and the guidance wall. The vertical air bubble channel keeps the air bubble carpet close to the filter screen so that the cleaning effect remains constant over the vertical height of the filter screen. Preferably, the guidance wall completely covers the corresponding filter screen, seen in horizontal direction. More preferably, the vertical guidance wall has a lower end edge being arranged vertically not above the air exit opening.

Preferably, the bubble generator housing is provided with an air inlet opening through which pressurized cleaning air is pumped by a suitable pump into the bubble generator housing. The lowest edge of the air inlet opening is at least 1.0 mm vertically above the top edge of the air exit opening. The wastewater surface level always remains vertically below of the air inlet opening so that the air inlet opening is safe against plugging caused by the wastewater substances.

Preferably, a non-return valve is provided upstream of the cleaning air inlet opening, and is, more preferably, arranged fluidically closer than 15 cm to the cleaning air inlet opening. The non-return valve opens in the flow direction of the cleaning air which is pumped to the bubble generator housing. Even if the cleaning air pump is not running, the static pressure of the air cushion in the bubble generator housing 41 remains relatively high so that the wastewater level remains below the cleaning air inlet opening.

According to independent claim 13, a process water sampling arrangement is provided comprising the process water sampling immersion probe with the features of one of the preceding claims. The process water sampling arrangement additionally comprises a cleaning air pump and an intermittent operation pump control intermittently activating and deactivating the cleaning air pump. The cleaning air pump is intermittently activated and stopped, for example due to a memorized cleaning schedule or due to a measured or determined height of the clogging structure in the exit openings. Since the cleaning air pump is not driven continuously, the lifetime of the cleaning air pump can considerately be extended.

One embodiment of the invention is described below with reference to the drawings, wherein
figure 1 schematically shows a process water analysis arrangement comprising a process water sampling immersion probe seen in vertical cross-section, and
figure 2 shows the process water sampling immersion probe of figure 1 in greater detail.

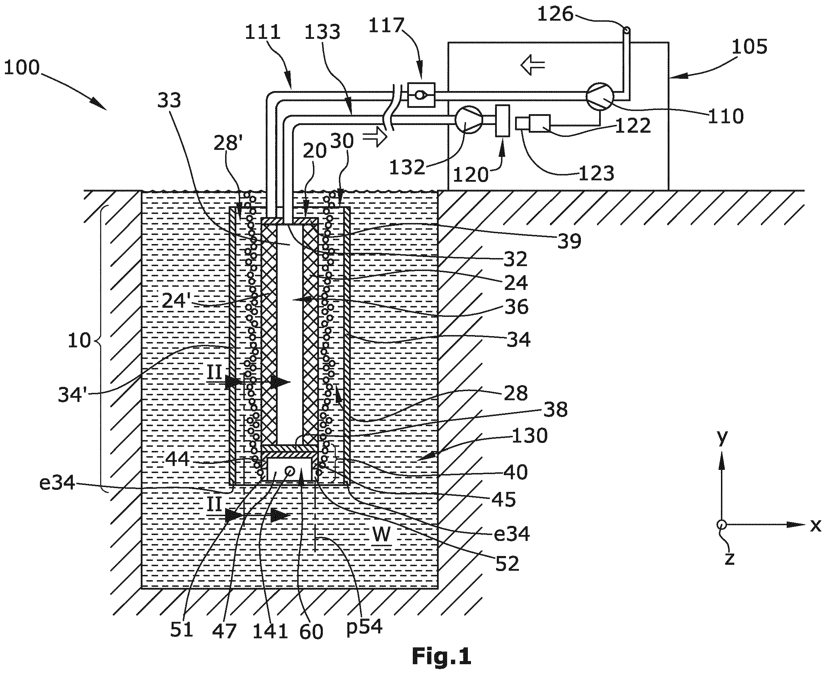

Figure 1 schematically shows a process water analysis arrangement 100 for continuously analyzing water samples of wastewater W. The wastewater W continuously flows into a wastewater tank 130 through a tank inlet, and continuously flows out of the wastewater tank 130 through a tank outlet, so that a general wastewater flow is generated within the wastewater tank 130. Alternatively, the wastewater tank 130 could be filled and emptied in intervals. The wastewater tank 130 can be a part of a wastewater treatment plant.

The process water analysis arrangement 100 is provided for quasi-continuously determining the concentration of one or more analyte of the wastewater W, for example of ammonium and/or phosphate.

The process water analysis arrangement 100 basically comprises a process water sampling immersion probe 10 which is completely immersed into the wastewater W and which is held in position by a stiff holding structure (not shown). The immersion probe 10 is fluidically, electrically and/or electronically connected to a land-based analyzer station 105 comprising an electric sample pump 132, an electric cleaning air pump 110, an electronic intermittent operation pump control 122 controlling the cleaning air pump 110, and an analyzer unit 120 for analyzing the concentration of one or more analyte of the water sample of the wastewater W.

The immersion probe 10 is generally provided as a flat, plane and rectangular body substantially comprising a filter module 20 with two parallel vertical filter screens 24, 24', a cleaning air generator 40 arranged vertically directly adjacent to the vertical lower end of the filter module 20, and a flow guidance arrangement 30 having two vertical guidance walls 34, 34'.

The two filter screens 24, 24' of the filter module 20 lie in a vertical plane yz, respectively, are parallel to each other and enclose, together with two vertical side walls 33, 33', a horizontal bottom wall 38 and a horizontal top wall 39, a rectangular filter module cavity 36. At the top end of the filter module cavity 36, the housing of the filter module 20 has a sample exit opening 32 through which the filtered water sample is continuously pumped by the sample pump 132 to the analyzer unit 120 via a sample line 133. The analyzer unit 120 quasi-continuously determines the concentration of the respective analyte of the water sample.

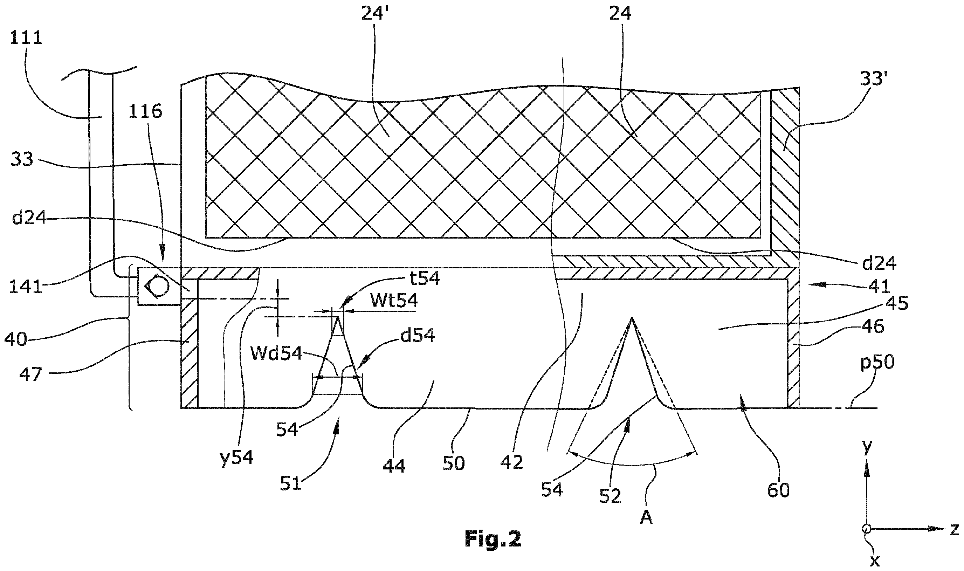

The cleaning air generator 40 is provided vertically close to the vertical lower end d24 of the two filter screens 24, 24' and is provided directly adjacent to the bottom wall 38 of the filter module housing. The cleaning air generator 40 comprises a substantially rectangular bubble generator housing 41 with in total four air exit openings 51, 52. The opening planes p54 of the air exit openings 51, 52 lie in a vertical plane yz, respectively and lie substantially in the same vertical planes as the corresponding filter screens 24, 24'. The rectangular bubble generator housing 41 is provided with a horizontal top wall 42, two vertical end walls 46, 47 being parallel to each other, and two vertical side walls 44, 45, each side wall 44, 45 comprising two of the air exit openings 51, 52, as shown in figure 2. The bubble generator housing 41 has an inverse cup-like structure, encloses a rectangular housing interior 60, has no bottom wall, and defines a horizontal housing bottom opening 50 having a horizontal housing opening plane p50.

One end wall 47 of the bubble generator housing 41 is provided with a cleaning air inlet opening 141 through which pressurized cleaning air is pumped into the bubble generator housing interior 60 when the cleaning air pump 110 is running. When the cleaning air pump 110 is running, cleaning air is sucked through a suction opening 126 and is pumped via a cleaning air line 111 to the cleaning air inlet opening 141. Two non-return valves 116, 117 are provided in the course of the cleaning air line 111, one non-return valve 116 being provided upstream of and adjacent to the cleaning air inlet opening 141, and the second non-return valve 117 downstream of and relatively close to the cleaning air pump 110. However, only one single non-return valve is necessary under normal circumstances, which is preferably the non-return verse 116 close to the cleaning air inlet opening 141.

Every vertical bubble generator housing side wall 44, 45 is provided with two air exit openings 51, 52, respectively. The air exit openings 51, 52 are designed to become horizontally continuously wider in the vertical downward direction, as shown in figure 2. The four air exit openings 51, 52 are identically shaped, and are substantially symmetrically inversely V-shaped with a total opening angle A of about 50°. Every air exit opening 51, 52 has a vertical opening plane p54. The horizontal opening top width wt54 at the opening's top end t54 is smaller than the opening down width wd54 at the opening's lower end d54, as shown in figure 2. The edges 54 of the air exit openings 51, 52 transition into the horizontal opening edge of the horizontal housing bottom opening 50 of the bubble generator housing 41. As a result, the air exit openings 51, 52 are perfectly accessible in vertical direction y for manually cleaning the air exit openings 51, 52.

As shown in figure 2, the top ends or the top edges of the air exit openings 51, 52 have a vertical distance y54 of at least 2 cm below the lowest opening edge of the cleaning air inlet opening 141 of the bubble generator housing 41.

As shown in figure 1, the process water sampling immersion probe 10 is provided with a guidance arrangement 30 with two plane vertical guidance walls 34, 34' being parallel to each other and to the filter screens 24, 24'. Each guidance wall 34, 34' is horizontally spaced apart from the corresponding filter screen 24, 24' with a few centimeters thereby defining a vertical air bubble channel 28, 28' between the filter screen 24, 24' and the corresponding guiding wall 34, 34'. As shown in figure 1, the linear lower end edges e34 of both guidance walls 34, 34' are vertically not above the air exit openings 51, 52 but are minimally below the lower ends d54 of the air exit openings 51, 52.

The intermittent operation pump control 122 is provided with an operation schedule memory 123 memorizing an intermittent working schedule for the cleaning air pump 110. The intermittent operation pump control 122 controls the activity of the cleaning air pump 110 by switching the cleaning air pump 110 intermittently on and off, with an on-rate of, for example, 50% and an interval length of a complete on/off cycle of five minutes, for example. Alternatively or additionally, the activity of the cleaning air pump 110 can be controlled in a closed-loop control circuit including a clogging sensor for determining the clogging rate of the air exit openings 51, 52.

## Claims

1. A process water sampling immersion probe (10) comprising
a filter module (20) comprising a filter screen (24, 24') and
a cleaning air generator (40) arranged vertically adjacent to the vertical lower end (d24) of the filter screen (24, 24'),
wherein the cleaning air generator (40) comprises a bubble generator housing (41) with an air exit opening (51, 52) having a non-horizontal opening plane (p54),
wherein the air exit opening (51, 52) widens vertically downwardly so that the air exit opening top width (wt54) at the openings top end (t54) is smaller than the air exit opening down width (wd54) at the openings lower end (d54).

2. The process water sampling immersion probe (10) of claim 1, wherein the air exit opening (51, 52) continuously widens between the opening's top end (t54) and the opening's lower end (d54) of the air exit opening (51, 52).

3. The process water sampling immersion probe (10) of one of the preceding claims, wherein the air exit opening (51, 52) is substantially inverse V-shaped.

4. The process water sampling immersion probe (10) of claim 3, wherein the average opening angle (A) of the inverse V-shaped air exit opening (51, 52) is more than 15°, more preferably more than 30°.

5. The process water sampling immersion probe (10) of one of the preceding claims, wherein at least two air exit openings (51, 52) are assigned to one filter screen (24, 24').

6. The process water sampling immersion probe (10) of one of the preceding claims, wherein the bubble generator housing (41) has a cup-like structure with a housing opening (50) having a horizontal housing opening plane (p50) so that the air exit opening (51, 52) has no opening edge at its downward end.

7. The process water sampling immersion probe (10) of one of the preceding claims, wherein the bubble generator housing (41) comprises a substantially vertical side wall (44, 45) comprising the air exit opening (51, 52).

8. The process water sampling immersion probe (10) of one of the preceding claims, wherein the filter screen (24, 24') is arranged vertically.

9. The process water sampling immersion probe (10) of one of the preceding claims, wherein a flow guidance arrangement (30) with a vertical guidance wall (34, 34') is provided, whereas the guidance wall (34) is distally spaced apart from the corresponding filter screen (24, 24') thereby defining a vertical air bubble channel (28, 28') between the filter screen (24, 24') and the guidance wall (34, 34').

10. The process water sampling immersion probe (10) of claim 9, wherein the vertical guidance wall (34, 34') has a lower end edge (e34) being arranged vertically not above the air exit opening (51, 52).

11. The process water sampling immersion probe (10) of one of the preceding claims, wherein the top end of the air exit opening (51, 52) has a vertical distance (y54) of at least 1.0 mm below a cleaning air inlet opening (141) of the bubble generator housing (41).

12. The process water sampling immersion probe (10) of one of the preceding claims, wherein the cleaning air flows into the bubble generator housing (41) via a cleaning air inlet opening (141), whereas a non-return valve (116, 117) is provided upstream of the cleaning air inlet opening (141).

13. A process water sampling arrangement (100) comprising the process water sampling immersion probe (10) with the features of one of the preceding claims, additionally comprising a cleaning air pump (110) and an intermittent operation pump control (122) intermittently activating the cleaning air pump (110).

## Amended claims

### Amended claims in accordance with Rule 137(2) EPC.

1. A process water sampling immersion probe (10) comprising a filter module (20) comprising a filter screen (24, 24') and
a cleaning air generator (40) arranged vertically adjacent to the vertical lower end (d24) of the filter screen (24, 24'),
wherein the cleaning air generator (40) comprises a bubble generator housing (41) with an air exit opening (51, 52) having a non-horizontal opening plane (p54),
wherein the air exit opening (51, 52) widens vertically downwardly so that the air exit opening horizontal top width (wt54) at the openings top end (t54) is smaller than the air exit opening horizontal down width (wd54) at the openings lower end (d54).

2. The process water sampling immersion probe (10) of claim 1, wherein the air exit opening (51, 52) continuously widens between the opening's top end (t54) and the opening's lower end (d54) of the air exit opening (51, 52).

3. The process water sampling immersion probe (10) of one of the preceding claims, wherein the air exit opening (51, 52) is substantially inverse V-shaped.

4. The process water sampling immersion probe (10) of claim 3, wherein the average opening angle (A) of the inverse V-shaped air exit opening (51, 52) is more than 15°, more preferably more than 30°.

5. The process water sampling immersion probe (10) of one of the preceding claims, wherein at least two air exit openings (51, 52) are assigned to one filter screen (24, 24').

6. The process water sampling immersion probe (10) of one of the preceding claims, wherein the bubble generator housing (41) has a cup-like structure with a housing opening (50) having a horizontal housing opening plane (p50) so that the air exit opening (51, 52) has no opening edge at its downward end.

7. The process water sampling immersion probe (10) of one of the preceding claims, wherein the bubble generator housing (41) comprises a substantially vertical side wall (44, 45) comprising the air exit opening (51, 52).

8. The process water sampling immersion probe (10) of one of the preceding claims, wherein the filter screen (24, 24') is arranged vertically.

9. The process water sampling immersion probe (10) of one of the preceding claims, wherein a flow guidance arrangement (30) with a vertical guidance wall (34, 34') is provided, whereas the guidance wall (34) is distally spaced apart from the corresponding filter screen (24, 24') thereby defining a vertical air bubble channel (28, 28') between the filter screen (24, 24') and the guidance wall (34, 34').

10. The process water sampling immersion probe (10) of claim 9, wherein the vertical guidance wall (34, 34') has a lower end edge (e34) being arranged vertically not above the air exit opening (51, 52).

11. The process water sampling immersion probe (10) of one of the preceding claims, wherein the top end of the air exit opening (51, 52) has a vertical distance (y54) of at least 1.0 mm below a cleaning air inlet opening (141) of the bubble generator housing (41).

12. The process water sampling immersion probe (10) of one of the preceding claims, wherein the cleaning air flows into the bubble generator housing (41) via a cleaning air inlet opening (141), whereas a non-return valve (116, 117) is provided upstream of the cleaning air inlet opening (141).

13. A process water sampling arrangement (100) comprising the process water sampling immersion probe (10) with the features of one of the preceding claims, additionally comprising a cleaning air pump (110) and an intermittent operation pump control (122) intermittently activating the cleaning air pump (110).
